# EUROPEAN PATENT APPLICATION

(11) **EP 4 497 830 A1**
(43) Date of publication of application: **29.01.2025**
(21) Application number: 24190316.0
(22) Date of filing: 23.07.2024
(51) Int. Cl.: C12P 7/10, C12P 19/02, C12P 19/14

(54) **ONIUM SALT COMPOSITIONS, METHODS FOR PRODUCING CELLULOSE HYDROLYSATES, ALCOHOLS AND/OR ORGANIC ACIDS IN THE PRESENCE OF ONIUM SALT COMPOSITIONS**

(30) Priority: 27.07.2023 JP 2023122660; 22.05.2024 JP 2024083080
(71) Applicant: Koei Chemical Company, Limited, Sodegaura-shi, Chiba 299-0266 (JP)
(72) Inventor: Tamakoshi, Satomi, Sodegaura-shi, Chiba 299-0266 (JP)
(74) Representative: Müller-Boré & Partner Patentanwälte PartG mbB

(57) **Abstract**

Provided is a composition containing an onium salt that allows enzymatic fermentation and microbial fermentation to proceed successfully. The present invention provides an onium salt composition containing an onium salt, a cellulose-containing biomass, and an enzyme, the onium salt being represented by formula (1): 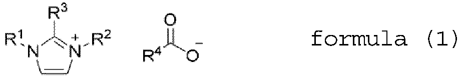 (wherein R¹ to R⁴ are as defined in the specification) .

## Description

### Technical Field

The present invention relates to an onium salt composition, a method for producing a cellulose hydrolysate in the presence of the onium salt composition, and a method for producing an alcohol and/or an organic acid in the presence of the onium salt composition.

### Background Art

Biomass derived from plants is expected to be used as an alternative to petroleum resources. Attempts have been made to utilize biomass for energy or other purposes such as various materials. In order to make effective use of biomass as an energy source, other raw materials, it is desirable to decompose and saccharify biomass into a carbon source that can be easily used by animals or microorganisms.

For such decomposition and saccharification, it is known to dissolve cellulose contained in biomass using an ionic liquid, degrade the cellulose into glucose by using an enzyme such as cellulase, and ferment the glucose using a microorganism to produce an alcohol and/or an organic acid.

For example, Patent Literature (PTL) 1 discloses a method for saccharifying cellulose, the method comprising treating a cellulose-containing material with an ionic liquid containing substituted pyridinium as a cation species and reacting the resulting treatment liquid with cellulose to saccharify cellulose; and a method for producing ethanol from a cellulose-containing material, the method further comprising mixing the reaction solution, which has been obtained by reacting the treatment liquid with cellulose, with an ethanol-producing microorganism and culturing the microorganism.

Further, as an ionic liquid capable of dissolving cellulose, Non-patent Literature (NPL) 1 discloses an ionic liquid containing dialkylimidazolium as a cation. Specifically, 1-ethyl-3-methylimidazolium acetate, 1-allyl-3-methylimidazolium chloride, 1-butyl-3-methylimidazolium chloride, and 1,3-dimethylimidazolium dialkylphosphate are described.

### Citation List

### Patent Literature

PTL 1: JP2013-135641A

### Non-patent Literature

NPL 1: Green Chemistry, 2015, vol. 17, pp. 694-714.

### Summary of Invention

### Technical Problem

The onium salt that is an ionic liquid described in the prior art documents inhibits both enzymatic reaction and microbial fermentation. Therefore, after cellulose contained in biomass is dissolved in an onium salt, which is an ionic liquid, the onium salt must be removed.

In view of this state of the prior art, an object of the present invention is to provide a composition containing an onium salt that allows both enzymatic reaction and microbial fermentation to proceed successfully. Another object of the present invention is to provide a method for producing a cellulose hydrolysate, the method comprising enzymatic hydrolysis of cellulose in the presence of an onium salt that allows both enzymatic reaction and microbial fermentation to proceed successfully. A further object of the present invention is to provide a method for producing an alcohol and/or an organic acid, the method comprising fermenting a cellulose hydrolysate with a microorganism in the presence of the onium salt.

### Solution to Problem

The present inventors conducted intensive research on an onium salt that allows both enzymatic reaction and microbial fermentation to proceed smoothly, and on enzymatic reaction and microbial fermentation in the presence of the onium salt, and have accomplished the present invention.

Specifically, the present invention provides the following [1] to [18].
[1] An onium salt composition comprising an onium salt, a cellulose-containing biomass, and an enzyme,
   the onium salt being represented by formula (1): 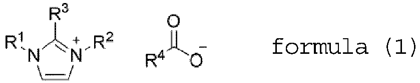
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond, and
   two or all of R¹ to R³ may be bound together with nitrogen and carbon atoms to which the R¹ to R³ are bound to form a cyclic structure, and
   R⁴ represents a hydrocarbon group optionally containing a heteroatom and may contain a branched and/or cyclic structure and/or a double bond.
[2] The onium salt composition according to [1], wherein the cellulose-containing biomass is dissolved in the onium salt represented by formula (1).
[3] The onium salt composition according to [1] or [2], further comprising water.
[4] The onium salt composition according to [3], wherein the onium salt represented by formula (1) has a concentration of 0.1 mass% or more and 20 mass% or less, based on the total amount of the onium salt composition taken as 100 mass%.
[5] An onium salt composition comprising an onium salt, glucose, and a microorganism,
   the onium salt being represented by formula (1): 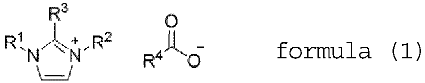
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond,
   two or all of R¹ to R³ may be bound together with nitrogen and carbon atoms to which the R¹ to R³ are bound to form a cyclic structure, and
   R⁴ represents a hydrocarbon group optionally containing a heteroatom and may contain a branched and/or cyclic structure and/or a double bond.
[6] The onium salt composition according to [5], wherein the glucose is dissolved in the onium salt represented by formula (1) .
[7] The onium salt composition according to [5] or [6], further comprising water.
[8] The onium salt composition according to [7], wherein the onium salt represented by formula (1) is present in a concentration of 0.1 mass% or more and 20 mass% or less, based on the total amount of the onium salt composition taken as 100 mass%.
[9] An onium salt composition comprising an onium salt, a cellulose-containing biomass, an enzyme, glucose, and a microorganism,
   the onium salt being represented by formula (1): 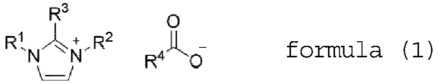
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond,
   two or all of R¹ to R³ may be bound together with nitrogen and carbon atoms to which the R¹ to R³ are bound to form a cyclic structure, and
   R⁴ represents a hydrocarbon group optionally containing a heteroatom and may contain a branched and/or cyclic structure and/or a double bond.
[10] The onium salt composition according to [9], further comprising water.
[11] The onium salt composition according to [10], wherein the onium salt represented by formula (1) is present in a concentration of 0.1 mass% or more and 20 mass% or less, based on the total amount of the onium salt composition taken as 100 mass%.
[12] A method for producing a cellulose hydrolysate, comprising hydrolyzing cellulose contained in a cellulose-containing biomass with an enzyme in the presence of an onium salt and water,
   the onium salt being represented by formula (1): 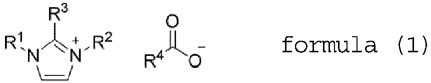
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond,
   two or all of R¹ to R³ may be bound together with nitrogen and carbon atoms to which the R¹ to R³ are bound to form a cyclic structure, and
   R⁴ represents a hydrocarbon group optionally containing a heteroatom and may contain a branched and/or cyclic structure and/or a double bond.
[13] The method for producing a cellulose hydrolysate according to [12], comprising the following steps (1) to (3):
   step (1): dissolving the cellulose-containing biomass in the presence of the onium salt represented by formula (1) to obtain a solution;
   step (2): adding the water and the enzyme to the solution obtained in step (1) to obtain a solution;
   step (3): stirring the solution obtained in step (2) and hydrolyzing cellulose contained in the cellulose-containing biomass with the enzyme to obtain a cellulose hydrolysate.
[14] A method for producing an alcohol and/or an organic acid, comprising fermenting a cellulose hydrolysate with a microorganism in the presence of an onium salt and water, the onium salt being represented by formula (1): 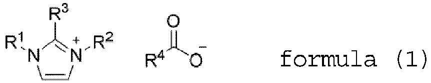
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond,
   two or all of R¹ to R³ may be bound together with the nitrogen and carbon atoms to which the R¹ to R³ are bound to form a cyclic structure, and
   R⁴ represents a hydrocarbon group optionally containing a heteroatom and may contain a branched and/or cyclic structure and/or a double bond.
[15] The method for producing an alcohol and/or an organic acid according to [14], comprising the following steps (1) to (3) :
   step (1): mixing the onium salt represented by formula (1), the cellulose hydrolysate, the water, and the microorganism to obtain a solution containing the microorganism;
   step (2): fermenting the cellulose hydrolysate with the microorganism in the solution obtained in step (1) to obtain a solution containing an alcohol and/or an organic acid; and
   step (3): separating the alcohol and/or the organic acid from the solution obtained in step (2).
[16] The method for producing an alcohol according to [14] or [15], wherein the alcohol is ethanol.
[17] A method for producing an alcohol and/or an organic acid from a cellulose-containing biomass in the presence of an onium salt and water,
   the onium salt being represented by formula (1): 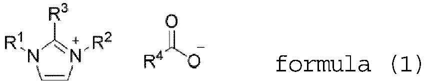
   wherein R¹ to R³ are identical or different,
   R¹ and R² each represent a C₂-C₁₂ hydrocarbon group containing 1 to 5 heteroatoms,
   R³ represents a hydrogen atom or a hydrocarbon group optionally containing a heteroatom,
   R¹ to R³ may contain a branched and/or cyclic structure and/or a double bond,
   two or all of R¹ to R³ may be bound together with nitrogen and carbon atoms to which the R¹ to R³ are bound to form a cyclic structure, and
   R⁴ represents a hydrocarbon group optionally containing a heteroatom and may contain a branched and/or cyclic structure and/or a double bond,
   the method comprising the following steps (1) to (6):
   step (1): dissolving the cellulose-containing biomass in the presence of the onium salt represented by formula (1) to obtain a solution;
   step (2): adding water and an enzyme to the solution obtained in step (1) to obtain a solution;
   step (3): stirring the solution obtained in step (2) and hydrolyzing cellulose contained in the cellulose-containing biomass with the enzyme to obtain a solution containing a cellulose hydrolysate;
   step (4): adding a microorganism to the solution obtained in step (3) to obtain a solution containing a microorganism;
   step (5): fermenting the cellulose hydrolysate with the microorganism in the solution obtained in step (4) to obtain a solution containing an alcohol and/or an organic acid; and step (6): separating the alcohol and/or the organic acid from the solution obtained in step (5).
[18] A method for recycling an onium salt, comprising
   recovering the solution containing the onium salt represented by formula (1) in the production method of [14] or [17], and
   separating the onium salt from the recovered solution to recycle the onium salt.

### Advantageous Effects of Invention

According to the present invention, a composition containing an onium salt that allows enzymatic reaction and microbial fermentation to proceed successfully can be provided. According to the present invention, there can also be provided a method for producing a cellulose hydrolysate, the method comprising enzymatic hydrolysis of cellulose in the presence of an onium salt that allows enzymatic reaction and microbial fermentation to proceed successfully. Further, according to the present invention, there can be provided a method for producing an alcohol and/or an organic acid, the method comprising fermenting a cellulose hydrolysate with a microorganism in the presence of the onium salt.

### Description of Embodiments

The present invention is explained below specifically. In one embodiment, the present invention provides an onium salt composition containing an onium salt represented by formula (1) below (referred to below as onium salt (1)), a cellulose-containing biomass, and an enzyme (referred to below as onium salt composition (1)).

Formula (1): 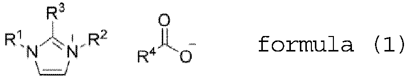 (wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond, two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond).

The "C₂-C₁₂ hydrocarbon group containing 1 to 5 heteroatoms" represented by R¹ and R² in formula (1) may be linear or may contain a branched and/or cyclic structure and/or a double bond. The number of heteroatoms is preferably 1 to 3 and more preferably 1 to 2. The number of carbon atoms in the hydrocarbon group is preferably 3 to 10 and more preferably 4 to 7.

Examples of the "heteroatoms" in R¹ and R² of formula (1) include a nitrogen atom, an oxygen atom, and a sulfur atom.

The "C₂-C₁₂ hydrocarbon group containing 1 to 5 heteroatoms" represented by R¹ and R² in formula (1) is one in which (i) a bond between two adjacent carbon atoms of a C₂-C₁₂ hydrocarbon group containing no heteroatoms and/or (ii) a hydrogen atom of one -CH₂- (a methylene group) and a hydrogen atom of another -CH₂- (a methylene group) of a C₂-C₁₂ hydrocarbon group containing no heteroatoms are substituted with a structure, such as -O-, -N<, -NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-. The number and combination of substitutions with these structures is not limited as long as the number of heteroatoms in R¹ and R² is 1 to 5.

The C₂-C₁₂ hydrocarbon group containing 1 to 5 heteroatoms is preferably one in which (i) a bond between two adjacent carbon atoms of a C₂-C₁₂ hydrocarbon group having no heteroatoms and/or (ii) a hydrogen atom of one -CH₂- (methylene group) and a hydrogen atom of another -CH₂- (a methylene group) of a C₂-C₁₂ hydrocarbon group having no heteroatoms are substituted with a structure of either -O- or -S-, and more preferably with a structure of -O-.

In the present invention, examples of the structure in which (i) a bond between two adjacent carbon atoms of a hydrocarbon group and/or (ii) a hydrogen atom of one -CH₂-(methylene group) and a hydrogen atom of another -CH₂- ((a methylene group) of a hydrocarbon group are substituted with a structure of either -O- or -S- include, in addition to structures such as ether structures and thioether structures, epoxy structures and thioepoxy structures, such as an epoxy group, a thioepoxy group, a glycidyl ether group, and an epoxycyclohexyl group. In this case, R¹ or R² may be an epoxy group, a thioepoxy group, or the like.

When two or more structures of -O- and/or -S- are present in R¹ and R², the number of carbon atoms between the structures is preferably 2 or more.

Two or more carbon atoms are preferably present between the nitrogen atom on the imidazole ring to which R¹ is bound and the heteroatom in R¹ that is the closest to the nitrogen atom on the imidazole ring. Two or more carbon atoms are preferably present between the nitrogen atom on the imidazole ring to which R² is bound and the heteroatom in R² that is the closest to the nitrogen atom on the imidazole ring.

In R¹ and R² of formula (1), the "C₂-C₁₂ hydrocarbon group containing no heteroatoms" may be linear or may contain a branched and/or cyclic structure and/or a double bond. Examples of the C₂-C₁₂ hydrocarbon group containing no heteroatoms include, but are not limited to, alkyl, alkenyl, alkynyl, cycloalkyl, cycloalkenyl, aryl, and aralkyl.

In R¹ and R² of formula (1), examples of the "C₂-C₁₂ hydrocarbon group containing 1 to 5 heteroatoms" include linear or branched C₂-C₁₂ alkyl groups containing 1 to 5 heteroatoms, linear or branched C₂-C₁₂ alkenyl groups containing 1 to 5 heteroatoms, linear or branched C₂-C₁₂ alkynyl groups containing 1 to 5 heteroatoms, linear or branched C₃-C₁₂ cycloalkyl groups containing 1 to 5 heteroatoms, linear or branched C₃-C₁₂ cycloalkenyl groups containing 1 to 5 heteroatoms, linear or branched C₆-C₁₂ aryl groups containing 1 to 5 heteroatoms, and optionally branched C₇-C₁₂ aralkyl groups containing 1 to 5 heteroatoms; and preferably linear or branched C₂-C₁₂ alkyl groups containing 1 to 5 heteroatoms, linear or branched C₂-C₁₂ alkenyl groups containing 1 to 5 heteroatoms, and linear or branched C₃-C₁₂ cycloalkyl groups containing 1 to 5 heteroatoms.

Preferable examples of "linear or branched C₂-C₁₂ alkyl groups containing 1 to 5 heteroatoms" include linear or branched C₃-C₁₀ alkyl groups containing 1 to 3 heteroatoms. In another embodiment, preferable examples are linear or branched C₃-C₁₀ alkyl groups containing 1 or 2 heteroatoms, and more preferably linear or branched C₄-C₇ alkyl groups containing 1 or 2 heteroatoms.

Preferable examples of "linear or branched C₂-C₁₂ alkenyl groups containing 1 to 5 heteroatoms" include linear or branched C₃-C₁₀ alkenyl groups containing 1 to 3 heteroatoms. In another embodiment, preferable examples are optionally branched C₃-C₁₀ cycloalkyl groups containing 1 to 2 heteroatoms, and more preferably linear or branched C₄-C₇ alkyl groups containing 1 or 2 heteroatoms.

Preferable examples of "linear or branched C₃-C₁₂ cycloalkyl groups containing 1 to 5 heteroatoms" include C₃-C₁₀ linear or branched cycloalkyl groups containing 1 to 3 heteroatoms. In another embodiment, the linear or branched C₃-C₁₂ cycloalkyl groups containing 1 to 5 heteroatoms is preferably linear or branched C₁-C₂ cycloalkyl groups containing 3 to 10 heteroatoms, and more preferably optionally branched C₄-C₇ cycloalkyl groups containing 1 to 2 heteroatoms.

In R¹ and R² of formula (1), specific examples of the C₂-C₁₂ hydrocarbon group containing no heteroatoms include ethyl, propyl, isopropyl, butyl, sec-butyl, isobutyl, tert-butyl, pentyl, neopentyl, hexyl, octyl, 2-ethylhexyl, decyl, dodecyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, and 2,4,6-trimethylphenyl. When (i) a bond between two adjacent carbon atoms of the C₂-C₁₂ hydrocarbon group containing no heteroatoms and/or (ii) a hydrogen atom of one -CH₂- (methylene) and a hydrogen atom of another -CH₂- (methylene) of the C₂-C₁₂ hydrocarbon group containing no heteroatoms are substituted with a structure, such as -O-, -N<, -NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-, a "C₂-C₁₂ hydrocarbon group containing 1 to 5 heteroatoms" is formed.

Specific examples of structures represented by R¹ and R² in formula (1) include, but are not limited to, 2-methoxyethyl, 2-ethoxyethyl, 2-propoxyethyl, 2-isopropoxyethyl, 2-phenoxyethyl, 2-(benzyloxy)ethyl, 2-(p-methoxybenzyloxy)ethyl, 2-(2-methoxyethoxymethoxy)ethyl, 2-(2-benzyloxyethoxy)ethyl, 2-(dimethylamino)ethyl, 3-methoxypropyl, 3-ethoxypropyl, 3-propoxypropyl, 3-allyloxypropyl, 3-isopropoxypropyl, 3-(1-methylpropoxy)propyl, 3-(2-methylpropoxy)propyl, 3-(1,1-dimethylethoxy)propyl, 3-butoxypropyl, 3-pentyloxypropyl, 3-neopentyloxypropyl, 3-hexyloxypropyl, 3-cyclohexyloxypropyl, 3-heptyloxypropyl, 3-octyloxypropyl, 3-nonyloxypropyl, 3-(2-ethylhexan-1-yl)oxypropyl, 3-(2,4,6-trimethylphenoxy)propyl, 3-(2-methoxyethoxy)propyl, 3-(dimethylamino)propyl, 4-methoxybutyl, 4-ethoxybutyl, 4-propoxybutyl, 4-isopropoxybutyl, 4-allyloxybutyl, 4-butoxybutyl, 4-(1-methylpropoxy)butyl, 4-(2-methylpropoxy)butyl, 4-(1,1-dimethylethoxy)butyl, 4-pentyloxybutyl, 4-neopentyloxybutyl, 4-hexyloxybutyl, 4-cyclohexyloxybutyl, 4-heptyloxybutyl, 4-octyloxybutyl, 4-(2-ethylhexyl-1-yl)oxybutyl, 5-methoxypentyl, 5-ethoxypentyl, 5-propoxypentyl, 5-isopropoxypentyl, 5-allyloxypentyl, 5-butoxypentyl, 5-(1-methylpropoxy)pentyl, 5-( 2-methylpropoxy)pentyl, 5-(1,1-dimethylethoxy)pentyl, 5-pentyloxypentyl, 5-neopentyloxypentyl, 5-hexyloxypentyl, 5-cyclohexyloxypentyl, 5-heptyloxypentyl, 6-methoxyhexyl, 6-ethoxyhexyl, 6-propoxyhexyl, 6-isopropoxyhexyl, 6-allyloxyhexyl, 6-butoxyhexyl, 6-(1-methylpropoxy)hexyl, 6-(2-methylpropoxy)hexyl, 6-(1,1-dimethylethoxy)hexyl, 6-pentyloxyhexyl, 6-neopentyloxyhexyl, 6-hexyloxyhexyl, 6-cyclohexyloxyhexyl, (2-methoxycyclohexan-1-yl)methyl, (2-ethoxycyclohexan-1-yl)methyl, (2-propoxycyclohexan-1-yl)methyl, (2-isopropoxycyclohexan-1-yl)methyl, (2-butoxycyclohexan-1-yl)methyl, [2-(1-methylpropoxy)cyclohexan-1-yl]methyl, [2-(2-methylpropoxy)cyclohexan-1-yl]methyl, [2-(1,1-dimethylethoxy)cyclohexan-1-yl]methyl, (2-pentyloxycyclohexan-1-yl)methyl, (2-neopentyloxycyclohexan-1-yl)methyl, 2-methylthioethyl, 3-methylthiopropyl, 1-(methoxymethyl)propyl, (oxolan-2-yl)methyl, (furan-2-yl)methyl, 2-[(furan-2-yl)methylthio]ethyl, 2-(methylsulfonyl)ethyl, (thiophen-2-yl)methyl, p-methoxyphenyl, p-ethoxyphenyl, 3,4-methylenedioxyphenyl, 3,4-methylenedioxybenzyl, and (7-oxabicyclo[2,2,1]heptan-2-yl)methyl; preferably 2-methoxyethyl, 3-methoxypropyl, 3-ethoxypropyl, 3-isopropoxypropyl, 3-butoxypropyl, 3-(2-methoxyethoxy)propyl, 3-methylthiopropyl, and 3-(dimethylamino)propyl; and more preferably 2-methoxyethyl, 3-methoxypropyl, and 3-(2-methoxyethoxy)propyl.

In formula (1), R¹ and R² are preferably identical.

In formula (1), R³ represents a hydrogen atom or a hydrocarbon group optionally containing a heteroatom. The "hydrocarbon group optionally containing a heteroatom" may be linear or may contain a branched and/or cyclic structure and/or a double bond. In R³, the hydrocarbon group may or may not contain a heteroatom.

When R³ in formula (1) is a hydrocarbon group containing no heteroatoms, the number of carbon atoms is preferably 1 to 12, more preferably 1 to 8, and even more preferably 1 to 3. When R³ is a hydrocarbon group containing a heteroatom, the number of carbon atoms is preferably 2 to 12, more preferably 2 to 8, and even more preferably 2 to 4.

When R³ in formula (1) contains a heteroatom, the number of heteroatoms is preferably 1 to 5, and more preferably 1 to 2. The number of carbon atoms is 2 or more, preferably 2 to 12, more preferably 2 to 8, and even more preferably 2 to 4.

When R³ in formula (1) is a hydrocarbon group containing a heteroatom, examples of the "heteroatom" include a nitrogen atom, an oxygen atom, and a sulfur atom.

When R³ is a hydrocarbon group containing a heteroatom, examples of the "heteroatom" include a nitrogen atom, an oxygen atom, and a sulfur atom. The number of heteroatoms is preferably 1 to 5 and more preferably 1 to 2.

In R³ of formula (1), the "hydrocarbon group containing a heteroatom" is one in which a bond between two adjacent carbon atoms of a hydrocarbon group containing no heteroatoms and/or (ii) a hydrogen atom of one -CH₂- (methylene) and a hydrogen atom of another -CH₂- (a methylene group) of a hydrocarbon group containing no heteroatoms are substituted with a structure, such as -O-, -N<, -NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-. The number and combination of substitutions by these structures are not particularly limited. However, the number of heteroatoms in R³ is preferably 1 to 5 heteroatoms and more preferably 1 to 2.

In R³ of formula (1), the "hydrocarbon group containing a heteroatom" is preferably one in which (i) a bond between two adjacent carbon atoms of a hydrocarbon group containing no heteroatoms and/or (ii) a hydrogen atom of one -CH₂- (methylene) and a hydrogen atom of another -CH₂- (a methylene group) of a hydrocarbon group containing no heteroatoms are substituted with a structure of either -0- or -S-, and more preferably a structure of -O-.

In the present invention, examples of the structure in which (i) a bond between two adjacent carbon atoms of a hydrocarbon group containing no heteroatoms and/or (ii) a hydrogen atom of one -CH₂- (methylene) of a hydrogen carbon group and a hydrogen atom of another -CH₂- (a methylene group) of a hydrocarbon group containing no heteroatoms is substituted with a structure of either -0- or -S- include, in addition to structures such as ether structures and thioether structures, epoxy structures and thioepoxy structures, such as an epoxy group, a thioepoxy group, a glycidyl ether group, and an epoxycyclohexyl group. In this case, R³ may be an epoxy group, a thioepoxy group, or the like.

When R³ contains two or more -O- or/and -S- structures, two or more carbon atoms are preferably present between the -O- or/and -S- structures.

In R³ of formula (1), examples of the "hydrocarbon group optionally containing a heteroatom" include linear or branched alkyl groups optionally containing a heteroatom, linear or branched alkenyl groups optionally containing a heteroatom, linear or branched alkynyl groups optionally containing a heteroatom, optionally branched cycloalkyl groups optionally containing a heteroatom, optionally branched cycloalkenyl groups optionally containing a heteroatom, optionally branched aryl groups optionally containing a heteroatom, and optionally branched aralkyl groups optionally containing a heteroatom. Preferable examples are linear or branched alkyl groups optionally containing a heteroatom, linear or branched alkenyl groups optionally containing a heteroatom, and optionally branched cycloalkyl groups optionally containing a heteroatom.

Preferable examples of the "linear or branched alkyl groups optionally containing a heteroatom" are linear or branched C₁-C₁₂ alkyl groups optionally containing 1 to 5 heteroatoms. In another embodiment, preferable examples are linear or branched C₁-C₁₂ alkyl groups optionally containing 1 or 2 heteroatoms, more preferably linear or branched C₁-C₈ alkyl groups optionally containing 1 to 2 heteroatoms, and even more preferably linear or branched C₁-C₄ alkyl groups optionally containing 1 to 2 heteroatoms. In another embodiment, preferable examples are linear or branched C₁-C₁₂ alkyl groups, more preferably linear or branched C₁-C₈ alkyl groups, and even more preferably linear or branched C₁-C₃ alkyl groups.

Preferable examples of the "linear or branched alkenyl groups optionally containing a heteroatom" are linear or branched C₂-C₁₂ alkenyl groups optionally containing 1 to 5 heteroatoms. In another embodiment, preferable examples are linear or branched C₂-C₁₂ alkenyl groups optionally containing 1 to 2 heteroatoms, more preferably linear or branched C₂-C₈ alkenyl groups optionally containing 1 to 2 heteroatoms, and even more preferably linear or branched C₂-C₄ alkenyl groups optionally containing 1 to 2 heteroatoms. In a further different embodiment, preferable examples are linear or branched C₂-C₁₂ alkenyl groups, more preferably linear or branched C₂-C₈ alkenyl groups, and even more preferably linear or branched C₂-C₃ alkenyl groups.

Preferable examples of "optionally branched cycloalkyl groups optionally containing a heteroatom" are optionally branched C₃-C₁₂ cycloalkyl groups optionally containing 1 to 5 heteroatoms. In another embodiment, preferable examples include optionally branched C₃-C₁₂ cycloalkyl groups optionally containing 1 to 2 heteroatoms, more preferably optionally branched C₃-C₈ cycloalkyl groups optionally containing 1 to 2 heteroatoms, and even more preferably optionally branched C₃-C₄ cycloalkyl groups optionally containing 1 to 2 heteroatoms. In a further different embodiment, preferable examples are optionally branched C₃-C₁₂ cycloalkyl groups, more preferably optionally branched C₃-C₈ cycloalkyl groups, and even more preferably a cycloalkyl group having 3 carbon atoms.

In R³ of formula (1), specific examples of the "hydrocarbon group containing no heteroatoms" include methyl, ethyl, propyl, isopropyl, butyl, sec-butyl, isobutyl, tert-butyl, pentyl, neopentyl, hexyl, octyl, 2-ethylhexyl, decyl, dodecyl, vinyl, ethynyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, styryl, and 2,4,6-trimethylphenyl; preferably methyl, ethyl, propyl, isopropyl, butyl, sec-butyl, isobutyl group, tert-butyl, pentyl, neopentyl, and hexyl; and more preferably methyl, ethyl, propyl, isopropyl, and tert-butyl.

In R³ of formula (1), preferable examples of the "hydrocarbon group containing a heteroatom" include, but are not limited to, 3-indolyl, p-methoxyphenyl, 7-oxabicyclo[2,2,1]hept-5-en-2-yl, 1,2-epoxyethyl, 1-methyl-2-methoxyvinyl, 2-furyl, 2-pyridyl, 4-imidazolyl, and benzyloxymethyl; and more preferably 1-methyl-2-methoxyvinyl and 2-furyl.

In formula (1), R³ is preferably a hydrogen atom or a methyl group, and more preferably a hydrogen atom.

In formula (1), two or all of R¹ to R³ may be bound together with the nitrogen and carbon atoms to which they are bound to form a cyclic structure. Specific examples of the cyclic structure include, but are not limited to, a ring structure formed by bonding R¹ and R², R¹ and R³, or R² and R³ together and being crosslinked with a C₂-C₂₄ hydrocarbon containing 2 to 10 heteroatoms.

In formula (1), R⁴ represents a hydrocarbon group, which may be linear or may contain a branched and/or cyclic structure and/or a double bond. R⁴ is preferably a C₁-C₁₂ hydrocarbon group, more preferably a C₁-C₈ hydrocarbon group, and even more preferably a C₁-C₃ hydrocarbon group.

Examples of the "hydrocarbon group" represented by R⁴ in formula (1) include alkyl, alkenyl, alkynyl, cycloalkyl, cycloalkenyl, aryl, and aralkyl. Preferable examples are linear or branched Cₗ-C₁₂ alkyl groups, linear or branched C₁-C₁₂ alkenyl groups, linear or branched C₁-C₁₂ alkynyl groups, optionally branched C₃-C₁₂ cycloalkyl groups, optionally branched C₃-C₁₂ cycloalkenyl groups, optionally branched C₆-C₁₂ aryl groups, and optionally branched C₇-C₁₂ aralkyl groups; and more preferably linear or branched C₁-C₃ alkyl groups, and linear or branched C₁-C₃ alkenyl groups.

Specific examples of the "hydrocarbon group" represented by R⁴ in formula (1) include, but are not limited to, methyl, ethyl, propyl, isopropyl, butyl, sec-butyl, isobutyl, tert-butyl, pentyl, neopentyl, hexyl, heptyl, octyl, 2-ethylhexyl, nonyl, decyl, undecyl, dodecyl, vinyl, ethynyl, propenyl, isopropenyl, allyl, propargyl, 1-propynyl, butenyl, crotyl, butadienyl, pentadienyl, cyclopropyl, cyclobutyl, cyclopentyl, cyclohexyl, norbornyl, adamantyl, phenyl, benzyl, tolyl, styryl, 2,4,6-trimethylphenyl, naphthyl, and 2,6-dimethyl-1,5-heptadienyl.

Specific examples of the anion represented by R⁴-CO-O⁻ in formula (1) include, but are not limited to, acetate ion, propionate ion, acrylate ion, propargylate ion, butyrate ion, isobutyrate ion, methacrylate ion, crotonate ion, tetrolate ion, cyclopropane carboxylate ion, valerate ion, isovalerate ion, pivalate ion, angelate ion, tiglate ion, cyclobutane carboxylate ion, caproate ion, cyclopentane carboxylate ion, enanthate ion, sorbate ion, cyclohexane carboxylate ion, benzoate ion, caprylate ion, 2-ethylhexyl carboxylate ion, toluate ion, 2-norbornane carboxylate ion, pelargonate ion, cinnamate ion, caprate ion, adamantane carboxylate ion, geranate ion, undecylate ion, laurate ion, and naphthalene carboxylate ion; preferably acetate ion, propionate ion, acrylate ion, propargylate ion, butyrate ion, isobutyrate ion, methacrylate ion, crotonate ion, tetrolate ion, valerate ion, isovalerate ion, and pivalate ion; and particularly preferably acetate ion, acrylate ion, and methacrylate ion.

Specific examples of the imidazolium cation of an onium salt represented by formula (1) include, but are not limited to, 1,3-bis(2-methoxyethyl)imidazolium, 1,3-bis(2-ethoxyethyl)imidazolium, 1,3-bis(2-propoxyethyl)imidazolium, 1,3-bis(2-isopropoxyethyl)imidazolium, 1,3-bis(2-phenoxyethyl)imidazolium, 1,3-bis(2-benzyloxyethyl)imidazolium, 1,3-bis[2-(p-methoxybenzyloxy)ethyl]imidazolium, 1,3-bis[2-(2-methoxyethoxymethoxy)ethyl]imidazolium, 1,3-bis[2-(2-benzyloxyethoxy)ethyl]imidazolium, 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis(3-pentyloxypropyl)imidazolium, 1,3-bis(3-neopentyloxypropyl)imidazolium, 1,3-bis(3-hexyloxypropyl)imidazolium, 1,3-bis(3-cyclohexyloxypropyl)imidazolium, 1,3-bis(3-heptyloxypropyl)imidazolium, 1,3-bis(3-octyloxypropyl)imidazolium, 1,3-bis(3-nonyloxypropyl)imidazolium, 1,3-bis{3-[(2-ethylhexyl)oxy]propyl}imidazolium, 1,3-bis[3-(2,4,6-trimethylphenoxy)propyl]imidazolium, 1,3-bis[3-(2-methoxyethoxy)propyl]imidazolium, 1,3-bis[2-(dimethylamino)ethyl]imidazolium, 1,3-bis[3-(dimethylamino)propyl]imidazolium, 1,3-bis(2-methylthioethyl)imidazolium, 1,3-bis{2-[(furan-2-yl)methylthio]ethyl]imidazolium, 1,3-bis[2-(furan-2-yl)methyl]imidazolium, 1,3-bis[1-(methoxymethyl)propyl]imidazolium, 1,3-bis(4-methoxybutyl)imidazolium, 1,3-bis(4-ethoxybutyl)imidazolium, 1,3-bis(4-propoxybutyl)imidazolium, 1,3-bis(4-isopropoxybutyl)imidazolium, 1,3-bis(4-allyloxybutyl)imidazolium, 1,3-bis(4-butoxybutyl)imidazolium, 1,3-bis[4-(1-methylpropoxy)butyl]imidazolium, 1,3-bis[4-(2-methylpropoxy)butyl]imidazolium, 1,3-bis[4-(1,1-dimethylethoxy)butyl]imidazolium, 1,3-bis(4-pentyloxybutyl)imidazolium, 1,3-bis(4-neopentyloxybutyl)imidazolium, 1,3-bis(4-hexyloxybutyl)imidazolium, 1,3-bis(4-cyclohexyloxybutyl)imidazolium, 1,3-bis(4-heptyloxybutyl)imidazolium, 1,3-bis(4-octyloxybutyl)imidazolium, 1,3-bis{4-[(2-ethylhexyl)oxy]butyl}imidazolium, 1,3-bis(5-methoxypentyl)imidazolium, 1,3-bis(5-ethoxypentyl)imidazolium, 1,3-bis(5-propoxypentyl)imidazolium, 1,3-bis(5-isopropoxypentyl)imidazolium, 1,3-bis(5-allyloxypentyl)imidazolium, 1,3-bis(5-butoxypentyl)imidazolium, 1,3-bis[5-(1-methylpropoxy)pentyl]imidazolium, 1,3-bis[5-(2-methylpropoxy)pentyl]imidazolium, 1,3-bis[5-(1,1-dimethylethoxy)pentyl]imidazolium, 1,3-bis(5-pentyloxypentyl)imidazolium, 1,3-bis(5-neopentyloxypentyl)imidazolium, 1,3-bis(5-hexyloxypentyl)imidazolium, 1,3-bis(5-cyclohexyloxypentyl)imidazolium, 1,3-bis(5-heptyloxypentyl)imidazolium, 1,3-bis(6-methoxyhexyl)imidazolium, 1,3-bis(6-ethoxyhexyl)imidazolium, 1,3-bis(6-propoxyhexyl)imidazolium, 1,3-bis(6-isopropoxyhexyl)imidazolium, 1,3-bis(6-allyloxyhexyl)imidazolium, 1,3-bis(6-butoxyhexyl)imidazolium, 1,3-bis[6-(1-methylpropoxy)hexyl]imidazolium, 1,3-bis[6-(2-methylpropoxy)hexyl]imidazolium, 1,3-bis[6-(1,1-dimethylethoxy)hexyl]imidazolium, 1,3-bis(6-pentyloxyhexyl)imidazolium, 1,3-bis(6-neopentyloxyhexyl)imidazolium, 1,3-bis(6-hexyloxyhexyl)imidazolium, 1,3-bis(6-cyclohexyloxyhexyl)imidazolium, 1,3-bis[(2-methoxycyclohexan-1-yl)methyl]imidazolium, 1,3-bis[(2-ethoxycyclohexan-1-yl)methyl]imidazolium, 1,3-bis[(2-propoxycyclohexan-1-yl)methyl]imidazolium, 1,3-bis[(2-isopropoxycyclohexan-1-yl)methyl]imidazolium, 1,3-bis[(2-butoxycyclohexan-1-yl)methyl]imidazolium, 1,3-bis{[2-(1-methylpropoxy)cyclohexan-1-yl]methyl}imidazolium, 1,3-bis{[2-(2-methylpropoxy)cyclohexan-1-yl]methyl}imidazolium, 1,3-bis{[2-(1,1-dimethylethoxy)cyclohexan-1-yl]methyl}imidazolium, 1,3-bis[(2-pentyloxycyclohexan-1-yl)methyl]imidazolium, 1,3-bis[(2-neopentyloxycyclohexan-1-yl)methyl]imidazolium, 1,3-bis(3-methylthiopropyl)imidazolium, 1,3-bis[2-(methylsulfonyl)ethyl]imidazolium, 1,3-bis[(thiophen-2-yl)methyl]imidazolium, 1,3-bis(p-methoxyphenyl)imidazolium, 1,3-bis(p-ethoxyphenyl)imidazolium, 1,3-bis(3,4-methylenedioxyphenyl)imidazolium, 1,3-bis(3,4-methylenedioxybenzyl)imidazolium, 1,3-bis{(7-oxabicyclo[2,2,1]heptan-2-yl)methyl}imidazolium, 1-methoxypropyl-3-methylthiopropylimidazolium, 1,3-bis(3-methoxypropyl)-2-methylimidazolium, and 1,3-bis(2-methoxyethyl)-2-methylimidazolium. Preferable examples are 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis[3-(2-methoxyethoxy)propyl]imidazolium, 1,3-bis(3-methylthiopropyl)imidazolium, 1-methoxypropyl-3-methylthiopropylimidazolium, 1,3-bis(3-methoxypropyl)-2-methylimidazolium, 1,3-bis(2-methoxyethyl)-2-methylimidazolium, and more preferably 1,3-bis(3-methoxypropyl)imidazolium, 1,3-bis (3-ethoxypropyl)imidazolium, 1-methoxypropyl-3-methylthiopropylimidazolium, 1,3-bis[3-(2-methoxyethoxy)propyl]imidazolium, 1,3-bis(2-methoxyethyl)-2-methylimidazolium, and 1,3-bis(3-methoxypropyl)-2-methylimidazolium.

The onium salt represented by formula (1) is preferably an onium salt composed of combination of a cation and an anion, the cation being selected from the group consisting of 1,3-bis(2-methoxyethyl)imidazolium, 1,3-bis(3-methoxypropyl)imidazolium, 1,3-bis(3-ethoxypropyl)imidazolium, 1,3-bis(3-methylthiopropyl)imidazolium, 1,3-bis[3-(2-methoxyethoxy)propyl]imidazolium, 1-methoxypropyl-3-methylthiopropylimidazolium, 1,3-bis(2-methoxyethyl)-2-methylimidazolium, and 1,3-bis(3-methoxypropyl)-2-methylimidazolium, and the anion being selected from the group consisting of acetate ion, acrylate ion, and methacrylate ion.

More specific examples of the onium salt represented by formula (1) include, but are not limited to, 1,3-bis(2-methoxyethyl)imidazolium acetate, 1,3-bis(3-methoxypropyl)imidazolium acetate, 1,3-bis(3-methoxypropyl)imidazolium acrylate, 1,3-bis(3-methoxypropyl) imidazolium methacrylate, 1,3-bis(3-ethoxypropyl)imidazolium acetate, 1-methoxypropyl-3-methylthiopropylimidazolium acetate, 1,3-bis(3-methylthiopropyl)imidazolium acetate, 1,3-bis(2-methoxyethyl)-2-methylimidazolium acetate, 1,3-bis(2-methoxyethyl)-2-methylimidazolium acrylate, 1,3-bis(2-methoxyethyl)-2-methylimidazolium methacrylate, 1,3-bis(3-methoxypropyl)-2-methylimidazolium acetate, 1,3-bis(3-methoxypropyl)-2-methylimidazolium acrylate, 1,3-bis(3-methoxypropyl)-2-methylimidazolium methacrylate, 1,3-bis[3-(2-methoxyethoxy)propyl]imidazolium acetate, 1,3-bis[3-(2-methoxyethoxy)propyl]imidazolium acrylate, and 1,3-bis[3-(2-methoxyethoxy)propyl]imidazolium methacrylate; and preferably 1,3-bis(3-methoxypropyl)imidazolium acetate, 1,3-bis(3-methoxypropyl)imidazolium acrylate, and 1,3-bis(3-methoxypropyl)imidazolium methacrylate.

The onium salt (1) of the present invention can be obtained, for example, by the production method shown in Reaction Scheme 1 below (Production Method 1). Specifically, with reference to the method for producing 1,3-bis(3-methoxypropyl)imidazolium acetate shown in Production Example 1, a person skilled in the art would be able to easily produce the onium salt (1) of the present invention. 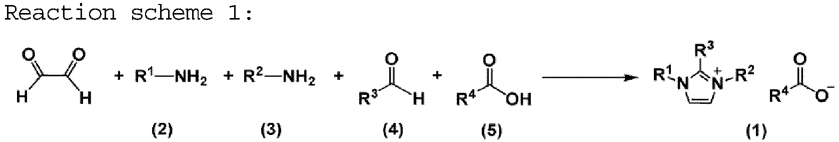 (wherein R¹, R², R³, and R⁴ each represent as defined above) .

Production Method 1 is described. Glyoxal, an amine compound represented by R¹-NH₂ (formula (2)) or R²-NH₂ (formula (3)), an aldehyde compound represented by R³-CHO (formula (4)), and a carboxylic acid represented by R⁴-COOH (formula (5)) are allowed to react.

Specific examples of the amine compound represented by R¹-NH₂ (formula (2)) or R²-NH₂ (formula (3)) include, but are not limited to, 2-methoxyethylamine, 2-ethoxyethylamine, 2-propoxyethylamine, 2-isopropoxyethylamine, 2-allyloxyethylamine, 2-butoxyethylamine, 2-(1-methylpropoxy)ethylamine, 2-(2-methylpropoxy)ethylamine, 2-(1,1-dimethylethoxy)ethylamine, 2-pentyloxyethylamine, 2-neopentyloxyethylamine, 2-hexyloxyethylamine, 2-phenoxyethylamine, 2-heptyloxyethylamine, 2-octyloxyethylamine, 2-nonyloxyethylamine, 2-decyloxyethylamine, 2-cyclopentyloxyethylamine, 2-cyclohexyloxyethylamine, 2-(2-ethylhexan-1-yl)oxyethylamine, 2-(2,4,6-trimethylphenoxy)ethylamine, 2-(benzyloxy)ethylamine, 2-(p-methoxybenzyloxy)ethylamine, 2-(2-methoxyethoxymethoxy)ethylamine, 2-(2-benzyloxyethoxy)ethylamine, 2-(dimethylamino)ethylamine, 3-methoxypropylamine, 3-ethoxypropylamine, 3-propoxypropylamine, 3-allyloxypropylamine, 3-isopropoxypropylamine, 3-(1-methylpropoxy)propylamine, 3-(2-methylpropoxy)propylamine, 3-(1,1-dimethylethoxy)propylamine, 3-butoxypropylamine, 3-pentyloxypropylamine, 3-neopentyloxypropylamine, 3-hexyloxypropylamine, 3-cyclohexyloxypropylamine, 3-heptyloxypropylamine, 3-octyloxypropylamine, 3-nonyloxypropylamine, 3-(2-ethylhexan-1-yl)oxypropylamine, 3-(2,4,6-trimethylphenoxy)propylamine, 3-(2-methoxyethoxy)propylamine, 3-(dimethylamino)propylamine, 4-methoxybutylamine, 4-ethoxybutylamine, 4-propoxybutylamine, 4-isopropoxybutylamine, 4-allyloxybutylamine, 4-butoxybutylamine, 4-(1-methylpropoxy)butylamine, 4-(2-methylpropoxy)butylamine, 4-(1,1-dimethylethoxy)butylamine, 4-pentyloxybutylamine, 4-neopentyloxybutylamine, 4-hexyloxybutylamine, 4-cyclohexyloxybutylamine, 4-heptyloxybutylamine, 4-octyloxybutylamine, 4-(2-ethylhexan-1-yl)oxybutylamine, 5-methoxypentylamine, 5-ethoxypentylamine, 5-propoxypentylamine, 5-isopropoxypentylamine, 5-allyloxypentylamine, 5-butoxypentylamine, 5-(1-methylpropoxy)pentylamine, 5-(2-methylpropoxy)pentylamine, 5-(1,1-dimethylethoxy)pentylamine, 5-pentyloxypentylamine, 5-neopentyloxypentylamine, 5-hexyloxypentylamine, 5-cyclohexyloxypentylamine, 5-heptyloxypentylamine, 6-methoxyhexylamine, 6-ethoxyhexylamine, 6-propoxyhexylamine, 6-isopropoxyhexylamine, 6-allyloxyhexylamine, 6-butoxyhexylamine, 6-(1-methylpropoxy)hexylamine, 6-(2-methylpropoxy)hexylamine, 6-(1,1-dimethylethoxy)hexylamine, 6-pentyloxyhexylamine, 6-neopentyloxyhexylamine, 6-hexyloxyhexylamine, 6-cyclohexyloxyhexylamine, 2-methoxycyclohexyl-1-ylmethylamine, (2-ethoxycyclohexan-1-yl)methylamine, (2-propoxycyclohexan-1-yl)methylamine, (2-isopropoxycyclohexan-1-yl)methylamine, (2-butoxycyclohexyl-1-yl)methylamine, [2-(1-methylpropoxy)cyclohexan-1-yl]methylamine, [2-(2-methylpropoxy)cyclohexan-1-yl]methylamine, [2-(1,1-dimethylethoxy)cyclohexan-1-yl]methylamine, (2-pentyloxycyclohexan-1-yl)methylamine, (2-neopentyloxycyclohexan-1-yl)methylamine, 2-methylthioethylamine, 3-methylthiopropylamine, 1-(methoxymethyl)propylamine, (oxolan-2-yl)methylamine, (furan-2-yl)methylamine, 2-[(furan-2-yl)methylthio]ethylamine, 2-(methylsulfonyl)ethylamine, (thiophen-2-yl)methylamine, p-methoxyphenylamine, p-ethoxyphenylamine, 3,4-methylenedioxyaniline, 3,4-methylenedioxybenzylamine, and (7-oxabicyclo[2,2,1]heptan-2-yl)methylamine; preferably 2-methoxyethylamine, 3-methoxypropylamine, 3-ethoxypropylamine, 3-isopropoxypropylamine, 3-butoxypropylamine, 3-(2-methoxyethoxy)propylamine, 3-methylthiopropylamine, and 3-(dimethylamino)propylamine; and more preferably 2-methoxyethylamine, 3-methoxypropylamine, and 3-(2-methoxyethoxy)propylamine. The amine compounds represented by R¹-NH₂ and R²-NH₂ may be identical or different.

Specific examples of the aldehyde compound represented by R³-CHO (formula (4)) include, but are not limited to, formaldehyde, acetaldehyde, propionaldehyde, butanal, pentanal, hexanal, heptanal, octanal, nonanal, decanal, acrolein, benzaldehyde, cinnamaldehyde, perylaldehyde, and vanillin; and preferably formaldehyde and acetaldehyde. As the formaldehyde, a cyclic oligomer or polymer capable of producing formaldehyde, such as paraformaldehyde, trioxane, or tetraoxane, in the reaction system can be used.

The aldehyde compound represented by R³-CHO (formula (4)), in the form of an aqueous solution or a solution in an alcohol, such as methanol, butanol, or 2-ethylhexanol, can be used as is.

Examples of the carboxylic acid represented by R⁴-COOH (formula (5)) include, but are not limited to, acetic acid, propionic acid, acrylic acid, propargylic acid, butyric acid, isobutyric acid, methacrylic acid, crotonic acid, tetrolic acid, cyclopropanecarboxylic acid, valeric acid, isovaleric acid, pivalic acid, angelic acid, tiglic acid, cyclobutanecarboxylic acid, caproic acid, cyclopentanecarboxylic acid, enanthic acid, sorbic acid, cyclohexanecarboxylic acid, benzoic acid, caprylic acid, 2-ethylhexylcarboxylic acid, toluic acid, 2-norbornanecarboxylic acid, pelargonic acid, cubanecarboxylic acid, cinnamic acid, capric acid, 3-noradamantanecarboxylic acid, geranic acid, undecylic acid, 1-naphthalenecarboxylic acid, 2-naphthalenecarboxylic acid, and 1-adamantanecarboxylic acid; preferably acetic acid, propionic acid, acrylic acid, propargylic acid, butyric acid, isobutyric acid, methacrylic acid, crotonic acid, tetrolic acid, valeric acid, isovaleric acid, and pivalic acid; and particularly preferably acetic acid, acrylic acid, and methacrylic acid.

Glyoxal, in the form of an aqueous solution or a solution in an alcohol, such as methanol, butanol, or 2-ethyl hexanol, can be used as is.

With respect to the amounts of the amine compounds represented by R¹-NH₂ (formula (2)) and R²-NH₂ (formula (3)), the total amount of R¹-NH₂ and R²-NH₂ is preferably 0.1 to 10 mol, and more preferably 0.5 to 3 mol, per mole of glyoxal. When R¹ and R² are different, the proportions of the two amine compounds (R¹-NH₂ and R²-NH₂) (molar ratio) are not particularly limited. The R¹-NH₂:R²-NH₂ ratio is in the range of 0:100 to 100:0. When the R¹-NH₂:R²-NH₂ ratio is either 0:100 or 100:0, R¹=R². When the R¹-NH₂:R²-NH₂ ratio is neither 0:100 nor 100:0, the onium salt (1) obtained by Production Method 1 is a mixture of compounds represented by the following formulas. The compounds represented by formula (1') and formula (1") below are both included within the scope of the onium salt represented by formula (1). The mixture of these compounds can be used as the onium salt composition described below. Optionally, the mixture can be purified to obtain a desired single compound and the single compound can be used as the onium salt (1).

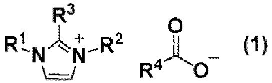

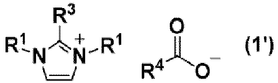

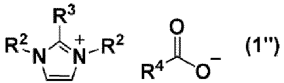

The aldehyde compound represented by R³-CHO (formula (4)), in the form of an aqueous solution or a solution in an alcohol, such as methanol, butanol, or 2-ethyl hexanol, can be used as is. The aldehyde compound is preferably used in an amount of 0.1 to 10 mol, and more preferably 0.5 to 5 mol, per mole of glyoxal.

The carboxylic acid represented by R⁴-COOH (formula (5)) is preferably used in an amount of 0.1 to 10 mol, and more preferably 0.5 to 2 mol, per mole of glyoxal.

In Production Method 1, a solvent may or may not be used. The solvent, if used, is not particularly limited as long as it does not affect the reaction. Specific examples of the solvent include aromatic hydrocarbon solvents such as toluene, benzene, and xylene; hydrocarbon solvents such as methylcyclohexane, cyclohexane, hexane, heptane, and octane; halogenated hydrocarbon solvents such as dichloromethane and chloroform; ether solvents such as diethyl ether, tetrahydrofuran, and 1,4-dioxane; alcohol solvents such as methanol, ethanol, and 2-propanol; N,N-dimethylformamide, acetonitrile, and water. Preferable solvents are alcohol solvents and water.

The onium salt (1) prepared beforehand can also be used as a reaction solvent in Preparation Method 1. The solvent can be used in the form of a mixture of two or more solvents freely selected. The solvent is preferably used in an amount of 50 parts by mass or less, and more preferably 0.1 to 10 parts by mass, per part by mass of glyoxal.

In Production Method 1, the reaction can be performed in an atmosphere of an inert gas that does not affect the reaction, such as nitrogen, argon, or helium.

When Production Method 1 is performed, the reaction pressure is usually atmospheric pressure. However, depending on the starting materials and/or solvent used, the reaction may be performed while the reaction system is pressurized to a pressure in the range of atmospheric pressure to 1 MPa.

The optimum reaction temperature may vary depending on, for example, the starting materials and solvent used, and is preferably -10°C or higher, and more preferably 0°C to 100°C.

After completion of the reaction in Production Method 1, the onium salt (1) may or may not be purified by a known purification method. Examples of the purification method include, but are not limited to, concentration, separation, recrystallization, dialysis, adsorption, and filtration.

The cellulose-containing biomass contained in the onium salt composition (1) is not particularly limited. Specific examples include natural fibers such as pulp, cotton, and hemp; recycled fibers such as rayon, cupra, and acetate; various straws such as rice straw; agricultural wastes such as bagasse and wood chips; and various wastes such as waste paper and construction waste. When biomass including, for example, natural fibers such as pulp, cotton, and hemp, recycled fibers such as rayon, cupra, and acetate, various straws such as rice straw, agricultural wastes such as bagasse and wood chips, and various wastes such as waste paper and construction waste, as mentioned above is dissolved in the onium salt (1), it is preferable to use cut pieces of such a material in order to shorten the time taken until the material has been dissolved.

In the present invention, cellulose refers to a polymer formed by polymerization of glucose through β-1,4-glucoside bonds, or a derivative thereof. The degree of polymerization of glucose in cellulose is not particularly limited, and is preferably 200 or more. Examples of such derivatives include derivatives obtained by carboxymethylation, aldehydization, or esterification. Cellulose may include cellooligosaccharides and cellobiose, which are partial degradation products of cellulose. Further, cellulose may be in the form of a glycoside, such as β-glucoside or lignocellulose, which is a complex of cellulose with lignin and/or hemicellulose, or a complex of cellulose with pectin or the like. The onium salt of the present invention is capable of dissolving cellulose at 10°C to 50°C, even if the cellulose is lignocellulose or even a complex with pectin or the like. The cellulose may be crystalline cellulose or non-crystalline cellulose, and is preferably crystalline cellulose. Further, the cellulose may be of natural origin or artificially synthesized. The origin of the cellulose is also not particularly limited. The cellulose may be derived from plants, fungi, or bacteria.

The enzyme contained in the onium salt composition (1) is a cellulose hydrolase. An example of the cellulose hydrolase is cellulase. Cellulase hydrolyzes β-1,4-glucosidic bonds of cellulose. Representative examples of the cellulase include, but are not limited to, endo-β-1,4-glucanase (simply referred to below as "endoglucanase"), cellobiohydrolase, and β-glucosidase.

In the present specification, cellulose hydrolysis means that β-1,4-glucosidic bonds of cellulose undergo hydrolysis to produce a reducing sugar, such as glucose or cellobiose. The cellulose hydrolysate is a reducing sugar produced by hydrolysis of cellulose, such as glucose or cellobiose.

Endoglucanase is an enzyme commonly referred to as cellulase. Endoglucanase cleaves cellulose from the inside of the molecule of cellulose to produce glucose, cellobiose, and cellooligosaccharides (the degree of polymerization of which is usually, but is not limited to, 3 or more and 10 or less). Endoglucanase is a representative example of an enzyme that hydrolyzes amorphous cellulose. Examples of the endoglucanase include, but are not limited to, endoglucanase derived from *Trichoderma reesei* (in particular, EGII).

Cellobiohydrolase degrades cellulose from either the reducing end or non-reducing end to release cellobiose. Cellobiohydrolase is a representative example of an enzyme that hydrolyzes crystalline cellulose. There are two types of cellobiohydrolase, which are called cellobiohydrolase 1 and cellobiohydrolase 2. Examples of the cellobiohydrolase include, but are not limited to, cellobiohydrolase derived from *Trichoderma reesei* (in particular, CBH2).

β-glucosidase is an exo-type hydrolase that cleaves glucose units from the non-reducing end of cellulose. β-Glucosidase cleaves the β1,4-glucosidic bond between an aglycone or sugar chain and β-D-glucose, and hydrolyzes cellobiose or cellooligosaccharide to produce glucose. β-Glucosidase is a representative example of an enzyme that hydrolyzes cellobiose or cellooligosaccharides. One type of β-glucosidase is called β-glucosidase 1. Examples of β-glucosidase include, but are not limited to, β-glucosidase derived from *Aspergillus aculeatus* (in particular, BGL1).

The cellulase may be of natural origin, may be produced by recombinant DNA technology, or may be a commercially available product. The enzyme may be purified or may be an enzyme-producing microorganism as is. The cellulase may be an enzyme preparation, a cellulase-expressing microorganism, or the like. Examples of the cellulase include cellulase derived from the genus *Clostridium,* such as *Clostridium thermocellum,* the genus *Trichoderma* such as *Trichoderma reesei,* or the genus *Aspergillus* such as *Aspergillus oryzae* and *Aspergillus niger.* The cellulase may be derived from hyperthermophilic archaea, such as those of the genus *Pyrococcus.*

The cellulase may be used in a combination of two or more. A combination capable of hydrolyzing cellulose into glucose is preferable.

The onium salt composition (1) is preferably an onium salt composition (1) in which a cellulose-containing biomass is dissolved in the onium salt (1).

The onium salt composition (1) preferably contains an onium salt (1), a cellulose-containing biomass, an enzyme, and water. More preferably, it is an onium salt composition (1) containing an onium salt (1), a cellulose-containing biomass, an enzyme, and water, wherein the cellulose-containing biomass is dissolved in the onium salt (1).

The onium salt composition (1) is preferably an onium salt composition (1) containing an onium salt (1), cellulose-containing biomass, an enzyme, and water, wherein the onium salt (1) is present in a concentration of 0.1 mass% or more and 20 mass% or less, based on the total amount of the onium salt composition (1) taken as 100 mass%. The concentration of the onium salt (1) is more preferably 0.5 mass% or more and 15 mass% or less, and even more preferably 1 mass% or more and 10 mass% or less.

One embodiment of the present invention relates to an onium salt composition containing an onium salt (1) represented by formula (1), glucose, and a microorganism (referred to below as onium salt composition (2)). The onium salt (1) contained in the onium salt composition (2) is the same as the onium salt (1) described above.

The glucose contained in the onium salt composition (2) is a monosaccharide molecule with a molecular formula of C₆H₁₂O₆, which constitutes cellulose.

The microorganism contained in the onium salt composition (2) is a microorganism that produces an alcohol and/or an organic acid by using glucose as a carbon source. Examples include ethanol-producing microorganisms. Ethanol-producing microorganisms causes fermentation to progress to thereby produce an organic acid, such as acetic acid, simultaneously with ethanol.

Examples of ethanol-producing microorganisms include, but are not limited to, yeast, ethanol-producing filamentous fungi, and ethanol-producing bacteria. Yeast is preferable. Examples of yeast include, but are not limited to, yeast of the genus *Saccharomyces,* such as *Saccharomyces cerevisiae,* yeast of the genus *Schizosaccharomyces,* such as *Schizosaccharomyces pombe,* yeasts of the genus *Candida,* such as *Candida krusei* and *Candidashehatae,* yeast of the genus *Pichia,* such as *Pichia pastoris* and *Pichia stipitis,* yeast of the genus *Hansenula,* yeasts of the genus *Trichosporon,* yeast of the genus *Brettanomyces,* yeast of the genus *Pachysolen,* yeast of the genus *Yamadazyma,* and yeast of the genus *Kluyveromyces,* such as *Kluyveromyces marxianus* and *Kluyveromyces lactis.* Among these, the yeast of the genus *Saccharomyces* is preferred and *Saccharomyces cerevisiae* is more preferred from the viewpoint of, for example, industrial availability. The yeast to be used can be fresh yeast or dry yeast. Examples of ethanol-producing filamentous fungi include, but are not limited to, filamentous fungi of the genus Mucor. Examples of ethanol-producing bacteria include, but are not limited to, *Zymomonas mobilis* and genetically modified *E*. *coli* that have been transformed to produce ethanol.

The onium salt composition (2) is preferably an onium salt composition (2) wherein glucose is dissolved in the onium salt (1).

The onium salt composition (2) preferably contains an onium salt (1), glucose, a microorganism, and water. More preferably, it is an onium salt composition (2) containing an onium salt (1), glucose, a microorganism, and water, wherein the glucose is dissolved in the onium salt (1).

The onium salt composition (2) is preferably an onium salt composition (2) containing an onium salt (1), glucose, a microorganism, and water, wherein the onium salt (1) is present in a concentration of 0.1 mass% or more and 20 mass% or less, based on the total amount of the onium salt composition (2) taken as 100 mass%. The concentration of the onium salt (1) is more preferably 0.5 mass% or more and 15 mass% or less, and even more preferably 1 mass% or more and 10 mass% or less.

One embodiment of the present invention relates to an onium salt composition containing an onium salt (1) represented by the above formula (1), a cellulose-containing biomass, an enzyme, glucose, and a microorganism (referred to below as the onium salt composition (3)). The onium salt (1), cellulose-containing biomass, enzyme, glucose, and microorganism contained in the onium salt composition (3) are the same as the onium salt (1), cellulose-containing biomass, enzyme, glucose, and microorganism described above, respectively.

The onium salt composition (3) preferably contains an onium salt (1), a cellulose-containing biomass, an enzyme, glucose, a microorganism, and water.

More preferably, it is an onium salt composition (3) containing an onium salt (1), a cellulose-containing biomass, an enzyme, glucose, a microorganism, and water, wherein the onium salt (1) is present in a concentration of 0.1 mass% or more and 20 mass% or less, based on the total amount of the onium salt composition (3) taken as 100 mass%. The concentration of the onium salt (1) is more preferably 0.5 mass% or more and 15 mass% or less, and even more preferably 1 mass% or less and 10 mass% or less.

One embodiment of the present invention relates to a method for producing a cellulose hydrolysate, the method comprising enzymatically hydrolyzing cellulose contained in cellulose-containing biomass in the presence of an onium salt (1) represented by formula (1) and water.

The method for producing a cellulose hydrolysate preferably comprises the following steps (1) to (3):
step (1): dissolving a cellulose-containing biomass in the presence of an onium salt represented by formula (1) to obtain a solution;
step (2): adding water and an enzyme to the solution obtained in step (1) to obtain a solution;
step (3): stirring the solution obtained in step (2) and hydrolyzing cellulose contained in the cellulose-containing biomass with an enzyme to obtain a cellulose hydrolysate.

With respect to the proportions of the onium salt (1) and the cellulose-containing biomass used in step (1) of the method for producing a cellulose hydrolysate, the content of the cellulose-containing biomass is preferably 1 to 30 mass%, more preferably 10 to 28 mass%, and particularly preferably 15 to 25 mass%, as calculated by the following formula: [{Cellulose-containing biomass (g)/(Cellulose-containing biomass + Onium salt (1))} × 100].

In the above step (1), the temperature at which the cellulose-containing biomass is dissolved in the presence of the onium salt (1) is preferably 0 to 120°C, more preferably 20 to 100°C, and particularly preferably 25 to 80°C.

The amount of water added in step (2) of the method for producing a cellulose hydrolysate is preferably 4 to 999 kg, more preferably 6 to 150 kg, and particularly preferably 19 to 99 kg, per kilogram of the solution obtained in step (1).

The amount of the enzyme added in step (2) is preferably 0.1 to 50 g, more preferably 1 to 10 g, and particularly preferably 2 to 5 g, per kilogram of the solution obtained in step (1).

The water and enzyme added in step (2) may be added separately or simultaneously. Alternatively, the water and enzyme may be mixed beforehand and the mixture may be added.

The temperature at which the water and enzyme are added in step (2) is preferably 10 to 60°C, and more preferably 25 to 50°C, in terms of temperature of the solution obtained in step (1) .

The enzymatic hydrolysis of cellulose in step (3) of the method for producing a cellulose hydrolysate is performed with stirring the solution obtained in step (2). The temperature at which the hydrolysis is performed is preferably 10 to 60°C, and more preferably 25 to 50°C. The hydrolysis time or stirring time is preferably 4 to 100 hours, more preferably 12 to 96 hours, and particularly preferably 24 to 72 hours.

After completion of step (3), the solution obtained in step (3) is filtered to separate the cellulose-containing biomass residue by filtration, and a solution containing cellulose hydrolysate can be obtained as a filtrate.

One embodiment of the present invention relates to a method for producing alcohol and/or an organic acid by fermenting a cellulose hydrolysate with a microorganism in the presence of an onium salt (1) represented by formula (1) and water.

The method for producing an alcohol and/or an organic acid preferably comprises the following steps (1) to (3):
step (1): mixing an onium salt represented by the above formula (1), a cellulose hydrolysate, water, and a microorganism;
step (2): fermenting the cellulose hydrolysate with the microorganism in the microorganism-containing solution obtained in step (1) to produce an alcohol and/or an organic acid; and
step (3): separating the alcohol and/or the organic acid from the alcohol- and/or organic acid-containing solution obtained in step (2) .

The method for producing an alcohol and/or an organic acid is preferably a method for producing an alcohol, and more preferably a method for producing ethanol.

The cellulose hydrolysate used in step (1) of the method for producing an alcohol and/or an organic acid comprising fermenting a cellulose hydrolysate with a microorganism can be a hydrolysate obtained by the method for producing a cellulose hydrolysate.

The proportions of the amounts of the onium salt, cellulose hydrolysate, water, and microorganism to be mixed in step (1) are 0.1 to 100 kg of the cellulose hydrolysate, 4 to 999 kg of the water, and 0.01 to 10 kg of the microorganism, per kg of the onium salt.

In step (1), the temperature at which the onium salt, cellulose hydrolysate, water, and microorganism are mixed is preferably 5 to 60°C, more preferably 10 to 50°C, and particularly preferably 25 to 40°C.

In step (2) of the method for producing an alcohol and/or an organic acid comprising fermenting a cellulose hydrolysate with a microorganism, the fermentation of the cellulose hydrolysate with the microorganism may be performed with stirring the solution containing the microorganism or by allowing the cellulose hydrolysate to stand (without stirring).

The temperature at which the cellulose hydrolysate is fermented with the microorganism in step (2) is preferably 5 to 60°C, more preferably 10 to 50°C, and particularly preferably 25 to 40°C.

The time of fermentation of the cellulose hydrolysate with the microorganism in step (2) is preferably 1 to 48 hours, more preferably 8 to 36 hours, and particularly preferably 12 to 24 hours.

In step (2), the cellulose hydrolysate is fermented with a microorganism to produce an alcohol and/or an organic acid. Examples of the alcohol produced include methanol and ethanol. Examples of the organic acid produced include formic acid and acetic acid.

In step (3) of the method for producing an alcohol and/or an organic acid comprising fermenting a cellulose hydrolysate with a microorganism, the separation of an alcohol and/or an organic acid can be performed by heating the solution containing an alcohol and/or an organic acid obtained in step (2) to evaporate the alcohol and/or the organic acid.

One embodiment of the present invention relates to a method for producing an alcohol and/or an organic acid from a cellulose-containing biomass in the presence of an onium salt (1) represented by formula (1) and water, the method comprising the following steps (1) to (6):
step (1): dissolving the cellulose-containing biomass in the presence of the onium salt (1) represented by formula (1) to obtain a solution;
step (2): adding water and an enzyme to the solution obtained in step (1) to obtain a solution;
step (3): stirring the solution obtained in step (2) and
hydrolyzing cellulose contained in the cellulose-containing biomass with an enzyme to obtain a solution containing a cellulose hydrolysate;
step (4): adding a microorganism to the solution obtained in step (3) to obtain a solution containing a microorganism;
step (5): fermenting the cellulose hydrolysate with the microorganism in the solution obtained in step (4) to obtain a solution containing an alcohol and/or an organic acid; and
step (6): separating the alcohol and/or the organic acid from the solution obtained in step (5).

With respect to the proportions of the onium salt (1) and the cellulose-containing biomass used in step (1) of the method for producing an alcohol and/or an organic acid from a cellulose-containing biomass, the content of the cellulose-containing biomass is preferably 1 to 30 mass%, more preferably 10 to 28 mass%, and particularly preferably 15 to 25 mass%, as calculated by the following formula: [{Cellulose-containing biomass (g)/(Cellulose-containing biomass + Onium salt (1))} × 100].

The temperature in step (1) is preferably 0 to 120°C, more preferably 20 to 100°C, and particularly preferably 25 to 80°C.

The amount of water added in step (2) of the method for producing an alcohol and/or an organic acid from a cellulose-containing biomass is preferably 4 to 999 kg, more preferably 6 to 150 kg, and particularly preferably 19 to 99 kg, per kilogram of the solution obtained in step (1).

The amount of the enzyme added in step (2) is preferably 0.1 to 50 g, more preferably 1 to 10 g, and particularly preferably 2 to 5 g, per kilogram of the solution obtained in step (1).

The water and enzyme to be added in step (2) may be added separately or simultaneously. Alternatively, the water and enzyme may be mixed beforehand and the mixture may be added.

The temperature at which the water and enzyme are added in step (2) is preferably 10 to 60°C, and more preferably 25 to 50°C, in terms of the temperature of the solution obtained in the step (1).

The enzymatic hydrolysis of cellulose in step (3) of the method for producing an alcohol and/or an organic acid from a cellulose-containing biomass is performed by stirring the solution obtained in step (2). The hydrolysis temperature is preferably 10 to 60°C and more preferably 25 to 50°C. The hydrolysis time or stirring time is preferably 4 to 100 hours, more preferably 12 to 96 hours, and particularly preferably 24 to 72 hours.

The amount of the microorganism added in step (4) of the method for producing an alcohol and/or an organic acid from a cellulose-containing biomass is preferably 0.01 to 10 g, and more preferably 0.1 to 5 g, per kilogram of the cellulose hydrolysate-containing solution obtained in step (3).

The temperature at which the microorganism is added in step (4) is preferably 5 to 60°C, more preferably 10 to 50°C, and particularly preferably 25 to 40°C, in terms of the temperature of the cellulose hydrolysate-containing solution obtained in step (3) .

The fermentation of the cellulose hydrolysate with a microorganism in step (5) of the method for producing an alcohol and/or an organic acid from a cellulose-containing biomass may be performed with stirring the microorganism-containing solution obtained in step (4) or by allowing the cellulose hydrolysate to stand (without stirring).

The temperature at which the cellulose hydrolysate is fermented with a microorganism in step (5) is particularly preferably 5 to 60°C, more preferably 10 to 50°C, and particularly preferably 25 to 40°C.

The time of fermentation of the cellulose hydrolysate with a microorganism in step (5) is preferably 1 to 48 hours, more preferably 8 to 36 hours, and particularly preferably 12 to 24 hours.

In step (5), the cellulose hydrolysate is fermented with a microorganism to thereby produce an alcohol and/or an organic acid. Examples of the alcohol produced include methanol and ethanol. Examples of the organic acid produced include formic acid and acetic acid.

The separation of an alcohol and/or an organic acid in step (6) of the method for producing an alcohol and/or an organic acid from a cellulose-containing biomass can be performed by heating the alcohol- and/or organic acid-containing solution obtained in step (5) and separating the alcohol and/or the organic acid by distillation.

One embodiment of the present invention relates to a method for recycling an onium salt in the method for producing an alcohol and/or an organic acid comprising fermenting a cellulose hydrolysate with a microorganism or in the method for producing an alcohol and/or an organic acid from a cellulose-containing biomass, the recycling method comprising recovering a solution containing an onium salt (1) represented by formula (1) and separating the onium salt (1) from the recovered solution to recycle the onium salt (1).

An alcohol and/or an organic acid is distilled and separated from the alcohol- and/or the organic acid-containing solution obtained in step (2) of the method for producing an alcohol and/or an organic acid comprising fermenting a cellulose hydrolysate with a microorganism to thereby recover an onium salt (1) or a liquid containing an onium salt (1). The onium salt (1) or liquid containing the onium salt (1) is recovered as a residue in the distillation of an alcohol and/or an organic acid.

The onium salt (1) or liquid containing the onium salt (1) can be recovered by distilling and separating the alcohol and/or the organic acid from the alcohol- and/or organic acid-containing solution obtained in step (5) of the method for producing an alcohol and/or an organic acid from a cellulose-containing biomass. The onium salt (1) or liquid containing the onium salt (1) is recovered as a residue in the distillation of the alcohol and/or the organic acid.

The recovered onium salt (1) or liquid containing the onium salt (1) can be recycled to the onium salt composition, the method for producing a cellulose hydrolysate, the method for producing an alcohol and/or an organic acid comprising fermenting a cellulose hydrolysate with a microorganism, or the method for producing an alcohol and/or an organic acid from a cellulose-containing biomass according to the present invention. Alternatively, the recovered onium salt (1) or liquid containing the onium salt (1) can also be used for other compositions, production methods, or applications using the onium salt.

### Examples

Next, the present invention is described in detail based on Examples. However, the present invention is not limited to these examples.

The 1,3-bis(3-methoxypropyl)imidazolium acetate ([(MeOPr)₂Im][AcO]) used in the Examples and Reference Examples was prepared as described in Preparation Example 1 below.

### Production Example 1

260 g (3.22 mol) of a 37 wt% formaldehyde aqueous solution (produced by Tokyo Chemical Industry Co., Ltd.), 290 g (4.83 mol) of acetic acid (produced by Junsei Chemical Co., Ltd.), 573 g (6.43 mol) of 3-methoxypropylamine (produced by Tokyo Chemical Industry Co., Ltd.), and 461 g (3.22 mol) of a 40 wt% glyoxal aqueous solution (produced by Tokyo Chemical Industry Co., Ltd.) were placed in a 5 L three-necked reactor purged with nitrogen. The resulting mixture was heated to 40°C and stirred for 3 hours to synthesize 811 g of 1,3-bis(3-methoxypropyl)imidazolium acetate ([(MeOPr)₂Im][AcO]) . The yield was 93%. The obtained ¹H NMR data of [(MeOPr) ₂Im][AcO] are as follows:
¹H NMR (DMSO-d₆) δ (ppm) = 9.41 (s, 1H), 7.78 (d, 2H), 4.22 (t, 4H), 3.33 (t, 4H), 3.22 (s, 6H), 2.07-2.00 (m, 4H), 1.56 (s, 3H)

The 1-ethyl-3-methylimidazolium acetate used in the Comparative Examples and Comparative Reference Examples is a product manufactured by Tokyo Chemical Industry Co., Ltd.

### Example 1

5.00 g of 1,3-bis(3-methoxypropyl)imidazolium acetate was placed in a flask and maintained at 50°C. 1.00 g of microcrystalline cellulose (Avicel (registered trademark) PH-101) was added at 50°C, and the resulting mixture was stirred until visual observation confirmed that microcrystalline cellulose had been completely dissolved. 95.0 g of ultrapure water and 10 µL of cellulase (produced by Sigma) were added, and the resulting mixture was stirred for 4 days. The remaining cellulose residue was recovered by filtration, and the glucose conversion rate was calculated. Table 1 shows the results.

### Comparative Example 1

The same procedure as in Example 1 was repeated except that 1-ethyl-3-methylimidazolium acetate was used in place of the 1,3-bis(3-methoxypropyl)imidazolium acetate used in Example 1. Table 1 shows the results.

### Example 2

0.20 g of 1,3-bis(3-methoxypropyl)imidazolium acetate, 0.5 g of glucose (β-D-glucose (including α-D-glucose), produced by Tokyo Chemical Industry Co., Ltd.), and 9.6 g of ultrapure water were placed in a sample tube. The resulting mixture was stirred until the glucose was completely dissolved. After the glucose had been completely dissolved, 0.05 g of dry yeast was added and the resulting mixture was allowed to stand overnight. After filtering off the insoluble matter, the amount of ethanol produced was quantified by GC (gas chromatography) to calculate the ethanol yield. Table 2 shows the results.

### Example 3

0.20 g of 1,3-bis(methoxyethoxypropyl)imidazolium acetate ([(MeOEtOPr)₂Im][AcO]), 0.5 g of glucose (β-D-glucose (including α-D-glucose), produced by Tokyo Chemical Industry Co., Ltd.), and 9.6 g of ultrapure water were placed in a sample tube. The resulting mixture was stirred until the glucose was completely dissolved. After the glucose had been completely dissolved, 0.05 g of dry yeast was added. The resulting mixture was allowed to stand overnight. After filtering off the insoluble matter, the amount of ethanol produced was quantified by GC (gas chromatography) to calculate the ethanol yield. Table 2 shows the results.

### Example 4

0.20 g of 1,3-bis(ethoxypropyl)imidazolium acetate ([(EtOPr)₂Im][AcO]), 0.5 g of glucose (β-D-glucose (including α-D-glucose), produced by Tokyo Chemical Industry Co., Ltd.), and 9.6 g of ultrapure water were placed in a sample tube. The resulting mixture was stirred until the glucose was completely dissolved. After the glucose had been completely dissolved, 0.05 g of dry yeast was added and the resulting mixture was allowed to stand overnight. After filtering off the insoluble matter, the amount of ethanol produced was quantified by GC (gas chromatography) to calculate the ethanol yield. Table 2 shows the results.

### Comparative Example 2

The same procedure as in Example 2 was repeated except that 1-ethyl-3-methylimidazolium acetate was used in place of the 1,3-bis(3-methoxypropyl)imidazolium acetate used in Example 2. Table 2 shows the results.

### Reference Example 1 and Comparative Reference Example 1

An antibacterial test (measurement of minimum inhibitory concentration (MIC value)) of 1,3-bis(3-methoxypropyl)imidazolium acetate and 1-ethyl-3-methylimidazolium acetate against yeast (*Saccharomyces cerevisiae*) was carried out. A lower MIC value indicates a greater degree of inhibition of enzymatic and/or microbial activity. Table 3 shows the results.

**Table 1**

| | Onium salt (1) | Residual cellulose (g) | Glucose conversion rate (%) |
|---|---|---|---|
| Example 1 | [MeOPr]₂Im][AcO] | 0.27 | 73 |
| Comparative Example 1 | 1-Ethyl-3-methylimidazolium acetate | 0.92 | 8 |

**Table 2**

| | Onium salt (1) | Ethanol yield (%) |
|---|---|---|
| Example 2 | [MeOPr]₂Im][AcO] | 66.64 |
| Example 3 | [MeOEtOPr]₂Im][AcO] | 53.77 |
| Example 4 | [EtOPr]₂Im][AcO] | 46.38 |
| Comparative Example 2 | 1-Ethyl-3-methylimidazolium acetate | 14.2 |

**Table 3**

| | Onium salt (1) | MIC value (%) |
|---|---|---|
| Reference Example 1 | [(MeOPr)₂Im[AcO] | 5 |
| Comparative Reference Example 1 | 1-Ethyl-3-methylimidazolium acetate | 0.5 |

### Example 5

2.00 g of 1,3-bis(3-methoxypropyl)imidazolium acetate was placed in a sample tube, and 0.50 g of microcrystalline cellulose (Avicel (registered trademark) PH-101) was added. The resulting mixture was heated and stirred at 80°C for 3 hours. After confirming by visual observation that the microcrystalline cellulose had been completely, 38.0 g of ultrapure water and 20 µL of cellulase (produced by Sigma) were added. The resulting mixture was stirred at 50°C for 10 hours. 0.50 g of dry yeast was then added and the resulting mixture was gently stirred at 30°C. After 7 hours, the insoluble matter was filtered off, and the amount of ethanol produced was quantified by GC (gas chromatography) to calculate the ethanol yield. Table 4 shows the results.

### Comparative Example 3

The same procedure as in Example 5 was repeated, except that 1-ethyl-3-methylimidazolium acetate was used in place of the 1,3-bis(3-methoxypropyl)imidazolium acetate used in Example 5. Table 4 shows the results.

### Comparative Example 4

The same procedure as in Example 5 was repeated, except that the 1,3-bis(3-methoxypropyl)imidazolium acetate used in Example 5 was not added and 2.00 g of ultrapure water was used in place of 2.00 g of 1,3-bis(3-methoxypropyl)imidazolium acetate. In this experiment, the microcrystalline cellulose did not dissolve. Table 4 shows the results.

**Table 4**

| | Onium salt (1) | Ethanol yield |
|---|---|---|
| Example 5 | [MeOPr]₂Im][AcO] | 51.5 |
| Comparative Example 3 | 1-Ethyl-3-methylimidazolium acetate | 16.0 |
| Comparative Example 4 | None | 27.3 |

### Industrial Applicability

According to the present invention, a composition containing an onium salt that allows enzymatic reaction and microbial fermentation to proceed successfully can be provided. According to the present invention, there is also provided a method for producing a cellulose hydrolysate, the method comprising enzymatic hydrolysis of cellulose in the presence of an onium salt that allows enzymatic reaction and microbial fermentation to proceed successively. Further, according to the present invention, there is provided a method for producing an alcohol and/or an organic acid, the method comprising fermenting a cellulose hydrolysate with a microorganism in the presence of the onium salt.

## Claims

1. An onium salt composition comprising an onium salt, a cellulose-containing biomass, and an enzyme,
the onium salt being represented by formula (1): 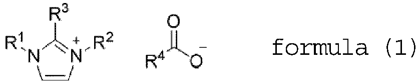
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond, and
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond.

2. The onium salt composition according to claim 1, wherein the cellulose-containing biomass is dissolved in the onium salt represented by formula (1).

3. The onium salt composition according to claim 1 or 2, further comprising water.

4. The onium salt composition according to claim 3, wherein the onium salt represented by formula (1) has a concentration of 0.1 mass% or more and 20 mass% or less, based on the total amount of the onium salt composition taken as 100 mass%.

5. An onium salt composition comprising an onium salt, glucose, and a microorganism,
the onium salt being represented by formula (1): 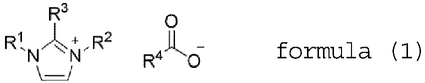
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond,
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond.

6. The onium salt composition according to claim 5, wherein the glucose is dissolved in the onium salt represented by formula (1).

7. The onium salt composition according to claim 5 or 6, further comprising water.

8. The onium salt composition according to claim 7, wherein the onium salt represented by formula (1) is present in a concentration of 0.1 mass% or more and 20 mass% or less, based on the total amount of the onium salt composition taken as 100 mass%.

9. An onium salt composition comprising an onium salt, a cellulose-containing biomass, an enzyme, glucose, and a microorganism,
the onium salt being represented by formula (1): 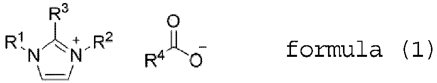
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond,
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond.

10. The onium salt composition according to claim 9, further comprising water.

11. The onium salt composition according to claim 10, wherein the onium salt represented by formula (1) is present in a concentration of 0.1 mass% or more and 20 mass% or less, based on the total amount of the onium salt composition taken as 100 mass%.

12. A method for producing a cellulose hydrolysate, comprising hydrolyzing cellulose contained in a cellulose-containing biomass with an enzyme in the presence of an onium salt and water,
the onium salt being represented by formula (1): 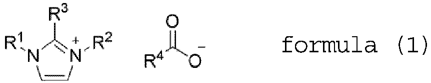
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond,
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond.

13. The method for producing a cellulose hydrolysate according to claim 12, comprising the following steps (1) to (3):
step (1): dissolving the cellulose-containing biomass in the presence of the onium salt represented by formula (1) to obtain a solution;
step (2): adding the water and the enzyme to the solution obtained in step (1) to obtain a solution;
step (3): stirring the solution obtained in step (2) and
hydrolyzing cellulose contained in the cellulose-containing biomass with the enzyme to obtain a cellulose hydrolysate.

14. A method for producing an alcohol and/or an organic acid, comprising fermenting a cellulose hydrolysate with a microorganism in the presence of an onium salt and water,
the onium salt being represented by formula (1): 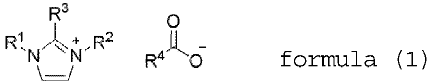
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond,
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond.

15. The method for producing an alcohol and/or an organic acid according to claim 14, comprising the following steps (1) to (3) :
step (1): mixing the onium salt represented by formula (1), the cellulose hydrolysate, the water, and the microorganism to obtain a solution containing the microorganism;
step (2): fermenting the cellulose hydrolysate with the microorganism in the solution obtained in step (1) to obtain a solution containing an alcohol and/or an organic acid; and
step (3): separating the alcohol and/or the organic acid from the solution obtained in step (2).

16. The method for producing an alcohol according to claim 14 or 15, wherein the alcohol is ethanol.

17. A method for producing an alcohol and/or an organic acid from a cellulose-containing biomass in the presence of an onium salt and water,
the onium salt being represented by formula (1): 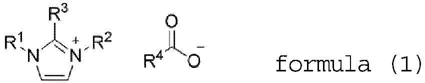
wherein R¹ to R³ are identical or different,
R¹ and R² each represent a C₂-C₁₂ hydrocarbon group containing 1 to 5 heteroatoms,
R³ represents a hydrogen atom or a hydrocarbon group optionally containing a heteroatom,
R¹ to R³ may contain a branched and/or cyclic structure and/or a double bond,
two or all of R¹ to R³ may be bound together with nitrogen and carbon atoms to which the R¹ to R³ are bound to form a cyclic structure, and
R⁴ represents a hydrocarbon group optionally containing a heteroatom and may contain a branched and/or cyclic structure and/or a double bond,
the method comprising the following steps (1) to (6):
step (1): dissolving the cellulose-containing biomass in the presence of the onium salt represented by formula (1) to obtain a solution;
step (2): adding water and an enzyme to the solution obtained in step (1) to obtain a solution;
step (3): stirring the solution obtained in step (2) and hydrolyzing cellulose contained in the cellulose-containing biomass with the enzyme to obtain a solution containing a cellulose hydrolysate;
step (4): adding a microorganism to the solution obtained in step (3) to obtain a solution containing a microorganism;
step (5): fermenting the cellulose hydrolysate with the microorganism in the solution obtained in step (4) to obtain a solution containing an alcohol and/or an organic acid; and
step (6): separating the alcohol and/or the organic acid from the solution obtained in step (5).

18. A method for recycling an onium salt, comprising
recovering the solution containing the onium salt represented by formula (1) in the production method of claim 14 or claim 17, and
separating the onium salt from the recovered solution to recycle the onium salt.
